## Europäisches Patentamt
## European Patent Office
## Office européen des brevets

(19)

(11) Publication number: **0 126 166**
**B1**

(12) # EUROPEAN PATENT SPECIFICATION

(45) Date of publication of patent specification: **31.01.90**

(51) Int. Cl.⁵: **C 12 N 15/00, C 12 P 21/00, C 12 N 1/20 // C12R1/19**

(21) Application number: **83104665.1**

(22) Date of filing: **11.05.83**

(54) Cloning vectors comprising a restriction site bank and the construction thereof.

(43) Date of publication of application:
**28.11.84 Bulletin 84/48**

(45) Publication of the grant of the patent:
**31.01.90 Bulletin 90/05**

(84) Designated Contracting States:
**AT BE CH DE FR GB IT LI LU NL SE**

(56) References cited:

GENE, vol. 8, 1979, pages 53-68, Elsevier/North-Holland Biomedical Press F.BRUNEL et al.: "Cloning of bacteriophage T5 DNA fragments in plasmid pBR322 and bacteriophage lambdagt WES"

GENE, vol. 16, 1981, pages 99-106, Elsevier/North-Holland Biomedical Press J.DAVISON et al.: "Cloning of bacteriophage T5 DNA fragments II. Isolation of recombinants carrying T5 PstI fragments"

GENE, vol. 16, 1981, pages 107-118, Elsevier/North-Holland Biomedical Press F.BRUNEL et al.: "Cloning of bateriophage T5

(73) Proprietor: **LABOFINA S.A.**
**52, rue de l'Industrie**
**B-1040 Bruxelles (BE)**

(72) Inventor: **Davison, John R.N.**
**27 Avenue Michel Steackmans**
**B-1200 Bruxelles (BE)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

(56) References cited:

DNA fragments III. Expression in Escherichia coli mini-cells"

MOLECULAR CLONING by T.MANIATIS, E.F. FRITSCH, J. SAMBROOK, C & H LABORATORY 1982,p.3-10

PALINDROMIC SEQUENCES OF RESTRICTION ENDONUCLEASES AND METHYLATES; Ed. by Boehringer Mannheim (1982), p.2-13

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to plasmid vectors containing a restriction site bank consisting of a small DNA segment comprising a large number of unique cloning sites and allowing the cloning of any foreign DNA material into prokaryotic and eukaryotic cells. The invention relates also to processes for the construction of plasmid vectors with restriction site banks and to the use of plasmid vectors containing such restriction site banks.

It is well known that restriction enzymes play a key role in recombinant DNA technology, e.g. by generating complementary cohesive ends from the cloning vector and from the DNA material to be cloned, thus allowing recombination between both. A large number of such restriction enzymes specific for well defined sites are now available, allowing great versatility in cloning experiments provided that the corresponding sites be present in the vector. Moreover, these sites should be present in limited number and preferably be unique so as to reduce as such as possible the number of recombination events necessary to obtain the desired recombinant DNA.

Previous concepts of plasmid vector design for cloning into prokaryotes have concentrated on the use of multiple antibiotic resistance genes containing unique restriction sites so that cloning at one of these sites results in sensitivity at one of the antibiotics, (insertional inactivation), thereby facilitating recombinant detection. While extremely useful, this concept has its limitations. Firstly it is difficult to construct a plasmid containing a large number of unique restriction sites since addition of other antibiotic resistance genes introduces duplicate sites and at the same time leads to an undesirable increase in size. Secondly, there are many situations in which insertional inactivation is not useful, e.g. when screening for specific rare recombinants in shotgun experiments, or when a suitable unique cloning site in an antibiotic resistance gene is not available. In the case of eukaryotes, the use of plasmid vectors with multiple antibiotic resistance genes is of little interest as eukaryotic cells are generally resistant to most antibiotics.

Because of these limitations, techniques have been developed which allow screening for hybrid colonies in other way, e.g. using hybridization probes, complementation of genetically marked host strains or simply by screening of a large number of plasmid DNA preparations using restriction enzymes. Similarly, the proportion of non-recombinant parental type plasmids can be reduced by alkaline phosphatase treatment of the vector prior to ligation or by cloning fragments between two different non-complementary restriction sites. For these reasons, the present invention has ignored insertional inactivation as a philosophy in plasmid construction and has evolved an alternative concept, that of the restriction site bank. In this specification the restriction site bank is defined as a segment of DNA specifically designed to have a small size and a maximum number of unique restriction sites. By this definition any sub-division or addition to a restriction site bank also results in a restriction site bank. According to the present invention, plasmid vectors are provided which contain a larger number of unique restriction sites than conventional vectors, preferably at least 10 restriction sites selected from the group consisting of EcoRI, ClaI, HindIII, EcoRV, BamHI, SphI, SalI(AccI, HincII), XmaIII, NruI, SacI, KpnI, AsuII, PstI, BclI, BglII, XbaI, PvuII, BalI, MstII, AvaI and XhoI. Thus, the vectors of the present invention allow the cloning of DNA material, e.g. complete genes, without running the risk of cleavage of the DNA material by the corresponding restriction enzyme. Furthermore, several genes can be cloned simultaneously.

Preferred embodiments of the present invention are described in detail below with reference to the drawings in which:

Figure 1 shows a schematic view of the replication segment of a vector according to the invention;

Figures 2a and b are schematic views of restriction site banks of the present invention;

Figure 3 gives a detailed view of a plasmid vector obtained in accordance with the process of the present invention; and

Figure 4 shows a schematic view of a yeast plasmid containing a restriction site bank of the present invention.

In accordance with a specific embodiment of the present invention, there is provided a plasmid vector for cloning any foreign DNA material in prokaryotic and eukaryotic orgnaisms, said plasmid vector containing a restriction site bank b1 derived from the HindIII-AvaI region of the bacteriophage T5 HindIII L fragment and comprising unique sites for the restriction enzymes HindIII, SacI, KpnI, AsuII, PstI, BglII, XbaI, HincII, PvuII, BalI, MstII and AvaI, arranged in the indicated order.

From this plasmid vector, other vectors can be derived by modification of the restriction site bank b1 hereabove described. Thus, the present invention also provides a plasmid vector wherein restriction sites for enzymes XmaIII and BclI have been added to bank b1 by replacing the 910 bp PstI-BglII segment thereof by the 210 bp PstI-BglII fragment from plasmid pKC7. The resulting restriction site bank referred to hereafter as b2, thus comprises unique cloning sites for restriction enzymes HindIII, SacI, KpnI, AsuII, PstI, XmaIII, BclI, BglII, XbaI, HincII, PvuII, BalI, MstII and AvaI, arranged in the indicated order.

The present invention further provides plasmid vectors derived from those described hereabove fusing the HindIII end of the restriction site banks b1 and b2 to the AvaI end of the EcoRI-AvaI fragment from plasmid pBR322, thus introducing additional cloning sites for the restriction enzymes EcoRI, ClaI, HindIII, EcoRV, BamHI, SphI, SalI (AccI, HincII), XmaIII and NruI arranged in the indicated order. The new restriction site banks thus constructed will be referred to hereafter as b3 or b4 according to whether it is derived from b1 or b2.

Restriction site banks b3 and b4 contained in these vectors comprise two HincII sites, one of which should preferably be absent. Accordingly, the present invention also provides plasmid vectors containing a modification of banks b3 or b4 comprising a unique HincII site from the pBR322-derived portion thereof. The restriction site bank thus modified will be referred to hereafter as b5 or b6 according to whether it is derived from b3 or b4.

Similarly, restriction site banks b4 and b6 comprise two XmaIII sites, one of which should also be absent. Thus, the present invention also provides plasmid vectors containing a modification of bank b4 or b6 comprising a unique XmaIII site from the pBR322-derived portion thereof. The restriction site banks thus modified will be referred to hereafter as b7 or b8 according whether it is derived from b4 or b6.

To further increase the number of useful restriction sites available for cloning there is still provided, in accordance with the present invention, plasmid vectors derived by introducing a XhoI site into the AvaI end of the restriction site bank of any of the previous vectors. In the case of restriction site bank b8, the new restriction site bank thus formed comprises the following cloning sites for restriction enzymes, EcoRI, ClaI, HindIII, EcoRV, BamHI, SphI, SalI(AccI, HincII), XmaIII, NruI, SacI, KpnI, AsuII, PstI, BclI, BglII, XbaI, PvuII, BalI, MstII, and XhoI, arranged in the indicated order.

To further increase the number of unique restriction sites available for cloning there is still provided, in accordance with the present invention, vectors wherein the ScaI site between the tet[R] gene and the SacI site is removed, thus leaving a unique ScaI site in the amp[R] gene.

In a further aspect, the present invention relates to processes for the construction of plasmid vectors with the restriction site banks described above, wherein the restriction site banks are fused with DNA fragments of special plasmids.

The process for the construction of restriction site bank b1 comprising unique cloning sites for the restriction enzymes HindIII, SacI, KpnI, AsuII, PstI, BglII, XbaI, HincII, PvuII, BalI, MstII and AvaI, arranged in the indicated order, comprises the steps of:

a) generating a single XbaI site by deleting the segment between the two XbaI sites of the HindIII-AvaI region of the bacteriophage T5 HindIII L fragment.

b) generating a single BglII site and simultaneously removing rendundant PvuII site by deleting the 1250 bp segment between the two BglII sites present in the same region.

From this first restriction site bank b1, further banks can be generated. For instance, restriction site bank b2 can be constructed in accordance with a process wherein excess DNA material present in restriction site bank b1 is removed by replacing the 910 bp PstI-BglII segment thereof by a 210 bp PstI-BglII fragment from plasmid pKC7, this resulting in the addition of sites for the restriction enzymes XmaIII and BclI.

Still other restriction site banks comprising more cloning sites than b1 and b2 can be constructed therefrom by fusion with a DNA fragment from plasmid pBR322. Thus, according to one embodiment of this construction process, the HindIII end of restriction site bank b1 is fused with the AvaI end of the EcoRI-AvaI fragment from plasmid pBR322. This fusion results in restriction site bank b3 comprising additional cloning sites for the restriction enzymes EcoRI, ClaI, HindIII, EcoRV, BamHI, SphI, SalI (AccI, HincII), SmaIII and NruI, arranged in the indicated order.

According to another embodiment of this construction process, the HindIII end of restriction site bank b2 is similarly fused with the AvaI end of the same fragment from pBR322, this fusion resulting in restriction site bank b4 containing the same additional cloning sites as in bank b3. The same restriction site bank b4 can also be constructed according to a process starting with restriction site bank b3 and wherein excess DNA material present in restriction site bank b1 is removed as described in the construction process of restriction site bank b2.

Restriction site bank b3 has two sites for the enzyme HincII: one in the pBR322-derived fragment and the other in the bacteriophage-derived region. From this restriction site bank, bank b5 can be constructed which contains a unique site for HincII. In accordance with the present invention, this is achieved by a process wherein the HincII site in the BglII-AvaI region of restriction site bank b1 is deleted while retaining the adjacent XbaI and PvuII sites, and then the resulting deleted region is substituted for the BglII-AvaI region of restriction site bank b3.

Similarly, restriction site bank b6 containing a unique HincII site can be constructed by substituting the same deleted region from bank b1 for the BglII-AvaI region of restriction site bank b4. The same restriction site bank b6 can also be constructed according to a process starting with restriction site bank b5 and wherein excess DNA material present in restriction site bank b1 is removed as described in the construction process of restriction site bank b2.

Restriction site bank b4 has two XmaIII sites: one in the pBR322-derived region and the other in the pKC7-derived fragment. According to another embodiment of the construction process of the present invention, the latter XmaIII site is deleted, this resulting in restriction site bank b7.

Similarly, the same XmaIII site can be deleted from restriction site bank b6, this resulting in restriction site bank b8 which contains unique sites for the restriction enzymes EcoRI, ClaI, HindIII, EcoRV, BamHI, SphI, SalI(AccI, HincII), XmaIII, NruI, SacI, KpnI, AsuII, PstI, BclI, BglII, XbaI, PvuII, BalI, MstII and AvaI, arranged in the indicated order.

The same restriction site bank b8 can also be constructed from bank 7 according to a process

wherein the *Hinc*II site in the *Bg/*II-*Ava*I region of restriction site bank b1 is deleted, and then the resulting deleted region is substituted for the *Bg/*II-*Ava*I region of restriction site bank b7.

From any of the preceding restriction site banks still further banks may be generated. For instance, in accordance with an embodiment of the construction process of the present invention, a *Xho*I site is fused to the *Ava*I end of any of the restriction site banks b1 to b8 constructed as described, thus destroying the original *Ava*I site (however *Ava*I also cleaves the new *Xho*I site).

It is an important practical aspect of the present invention that the restriction site banks constructed in accordance with the processes herein disclosed will be incorporated into special cloning vehicle whatever may be the host organism. Thus, they can be inserted into vector systems designed for cloning and expressing genes in prokaryotic organisms such as *Escherichia coli, Bacillus subtilis, Pseudomonas species*, etc...., or in eukaryotic organisms as yeasts, fungi and even animal or plant cells. In these different cases, the large number of unique restriction sites provides exceptional cloning versatility and the clustering thereof facilitates subsequent operations as subcloning and deletion analysis of large cloning fragments. Incorporated into vectors allowing the cloning of large fragments such as cosmids, they are especially useful for the construction of genomic libraries.

A useful method to take advantage of the plasmid vectors with the restriction site banks in accordance with the present invention is to incorporate the restriction site banks into a plasmid vector carrying an origin of replication recognized by the replication machinery of the selected host as well as a gene imparting resistance of the host to some drug, e.g. an antibiotic or a gene complementing some auxotrophy thereof for an essential metabolite, e.g. an amino acid, so as to allow efficient screening for the transformed organisms.

Most plasmids currently used for transforming prokaryotic organisms and more especially *E. coli*, can be used for this purpose. Examples of such plasmids are pBR322 and other related plasmids such as pBR327, pMB9, etc. Other plasmids such as the natural replicon *Col*.EI and other inrelated plasmids such as P15A, F, RSF1010, R616, etc... may also be used to design efficient vectors containing restriction site banks in accordance with this invention.

Similarly, plasmids used for the transformation of eukaryotic organisms may be used. Typical examples of such plasmids are those designed for the transformation of yeast, e.g. pJDB207, pJDB219, pMp78, etc...

In applying the stepwise process of the present invention for the construction of restriction site banks, the DNA fragment containing clustered cloning sites as obtained in each step must be produced and isolated in sufficient amount for being further processed. This can be accomplished by inserting said DNA fragment into a plasmid vector as those referred to hereabove and transforming a selected host with the resulting chimaeric plasmid. Although any microorganism able to be transformed by exogenous DNA can be used for this purpose, *E. coli* is a preferred host as well designed and efficient methods are available not only for the transformation thereof but also for DNA amplification and recovery. In the following examples, the strain *E. coli* MM294 is used as a host, unless otherwise indicated. The strain *E. coli* MM294 transformed with the Plasmid pJRD143 has been deposited at the Centraalbureau voor Schimelscultures, Baarn, Netherlands, on May 11, 1983 under file number LMD 83.07=CBS 396-83, and forms as such part of the invention.

In general, the currently available segments of DNA that permit replication and selection in the various organisms indicated above are not directly suitable for incorporation of the restriction site banks and require prior modification. A typical procedure in accordance with these requirements is given in the following examples which should be considered as illustrative only and not limitative of the present invention.

Example 1
Modification of the replication segment and selective marker

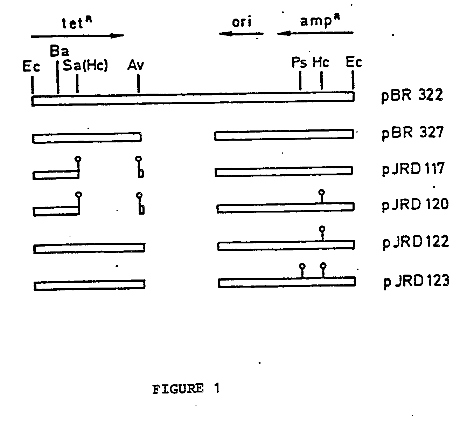

In this example, the vector used for the replication of the restriction site bank under construction is derived by modification of plasmid pBR327 obtained through *Eco*R II deletion from pBR322 (Soberon *et al.*, Gene 9, 1980, 287—305). The modification of pBR327 consisted in removing from the *amp*$^R$ gene the *Pst*I and *Hinc*II sites to allow unique *Pst*I and *Hinc*II sites present in the final restriction site bank to be used for cloning. (Failure to remove these sites would prevent cloning by inactivation of the *amp*$^R$ selective marker). The unique *Pvu*I and *Sca*I sites in the *amp*$^R$ gene were not removed since they are not provided in the final restriction site bank and because they may be useful to certain types of recombinant constructions. The procedure followed is shown in Figure 1. The *Hinc*II site in the *tet*$^R$ gene of pBR327 was removed by fusion between the unique *Sal*I and *Ava*I sites to give the tetracycline sensitive plasmid pJRD117. The now unique *Hinc*II site in the *amp*$^R$ gene was then removed by mutagenesis followed by repeated cycles of DNA isolation, restriction cleavage and transformation using the method of Talmadge and Gilbert (Gene 12, 1980, 235—241). This resulted in plasmid pJRD120 which has lost the unique *Hinc*II site while retaining *amp*$^R$. The region *Eco*RI-*Pst*I of pBR327 (co-ordinates 3271-2523) was then restored to pJRD120 to give *amp*$^R$-*tet*$^R$ plasmid pJRD122 which now has a unique *Hinc*II site. pJRD122 was then subjected to further mutagenesis and restriction-transformation cycles resulting in the selection of pJRD123 which is *amp*$^R$, *tet*$^R$ has a unique *Hinc*II site and is lacking a *Pst*I site.

The procedure resulted in an additional mutation causing a GC→AT base pair change at the position corresponding to co-ordinate 3132 of pBR322. This mutation has two important effects firstly it increases the copy number of the plasmid from about 30 to 120 so that a cloned gene would be better expressed due to increased gene dosage. Secondly it alters the normal incompatibility mechanism of the plasmid which is now able to co-exist side by side in the same cell as pBR322 and its derivatives (two pBR322 type plasmids cannot normally do this). An identical mutation has previously been independently described (Tomizawa and Itoh, Proc. Natl. Acad. Sc. USA 78, 1981, 6091—6100). The advantage of this is that it would be possible to introduce different genes cloned on different plasmids into the same cell.

Construction of the restriction site bank

The basis for the construction of the restriction site bank in accordance with this invention is the HindIII-L fragment from bacteriophage T5. This fragment contains restriction sites for SacI, KpnI, BgIII, XbaI and PvuII, none of these enzymes being able to cleave plasmid pBR327. However, it is too large (3.9 kb) for use in vector construction so that deletions are necessary to remove excess DNA and unwanted restriction sites.

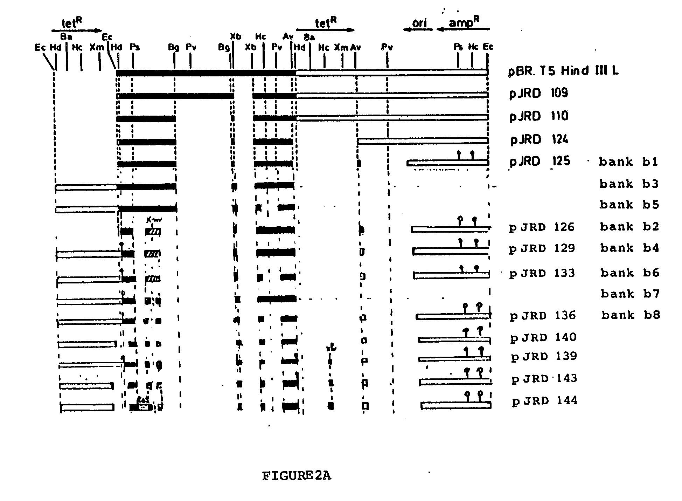

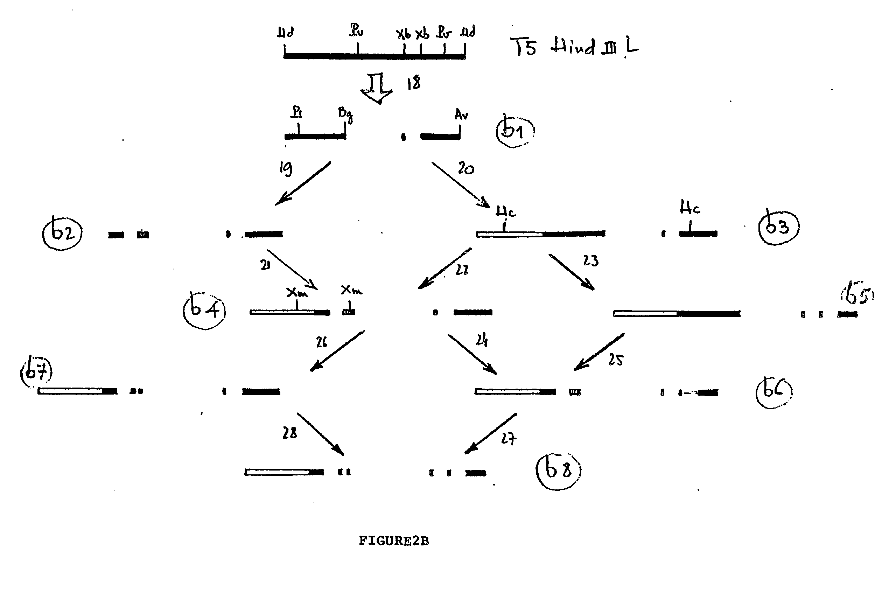

Figures 2a and b illustrate a typical procedure starting with the HindIII-L fragment from bacteriophage T5 initially inserted in plasmid vector pBR322 (Brunel et al., Gene 8, 1979, 53—68). Firstly, the 490 bp region between the two XbaI sites of said fragment was removed to generate a single XbaI site (Figure 2, pJRD109). Secondly, the 1250 bpBgIII-BgIII region was removed to generate a single BgIII and simultaneously delete an unwanted PvuII site (Figure 2, pJRD110). Thirdly, the 1405 bpAval-Aval fragment was deleted removing one of the two Aval sites of plasmid pJRD110, one of the two HindIII sites and the entire tet^R region (this latter being not expressed due to the insertion of the original HindIII-L fragment in the tet^R promoter), to give plasmid pJRD124 (Figure 2).

The region Aval-HindIII of the latter plasmid was then replaced by the corresponding region of the plasmid pJRD123 constructed as explained hereabove (Figure 1). This event simultaneously introduces the EcoRII deletion of pBR327, the amp^R region of pJRD123 carrying the Pst^o and Hinc^o mutations and the increased copy number mutation to give plasmid pJRD125 (Figure 2). The 910 bp PstI-BgIII region of this latter plasmid was then replaced by a 210 bp PstI-BgIII fragment from plasmid pKC7 (Rao & Rogers, Gene 7, 1979, 79—82), thus removing excess DNA and introducing a BcII and a XmaIII site, resulting in plasmid pJRD126 (Figure 2).

Plasmid pJRD126 is sensitive to tetracycline due to deletion of the entire HindIII-Aval region of pBR322 (coordinates 29—1424). This region contains several unique restriction sites that can be used for insertional inactivation and therefore can advantageously be included in a restriction site bank as considered in the present invention. The

said HindIII-Aval region was therefore re-introduced into pJRD126, resulting in plasmid pJRD129. To do this, the DNA of plasmid pBR322 was cleaved with Aval, repaired with DNA polymerase I Kleenow fragment and deoxynucleoside triphosphates and then cleaved with EcoRI. Simultaneously, the DNA of pJRD126 was cleaved with HindIII, repaired with DNA polymerase I Kleenow fragment and deoxynucleoside triphosphates and then cleaved with EcoRI. The two plasmids were then mixed and ligated, resulting in replacement of the EcoRI-HindIII region of pJRD126 by the EcoRI-Aval region of pBR322 by a cohesive-end-blunt-end ligation, the latter destroying both the HindIII site of pJRD126 and the Aval site of pBR322. The resulting plasmid pJRD129 is amp^R, tet^R and has one unique HindIII site and Aval site.

Plasmid pJRD129 has unique restriction sites for most restriction enzymes of interest but has two for HincII, one located in the tet^R region and the other in the 500 bp XbaI-PvuII fragment which contains no other sites of interest. This latter site and the adjacent excess DNA was removed by HincII digestion of pJRD126 (which has a unique HincII site) followed by digestion with nuclease Bal 31 and blunt-end ligation. The resulting plasmids were screened for retention of both the XbaI and PvuII sites and a deletion selected that retained only about 80 bp of the original XbaI-PvuII fragment (pJRD130). This deletion was then transferred to plasmid pJRD129 by digestion of both plasmids with BgIII+Aval followed by ligation and transformation, resulting in plasmid pJRD133.

Similarly, plasmid pJRD133 has two sites for restriction enzyme XmaIII, one located in the tet^R region and the other in the 210 bp PstI-BgIII region introduced from pKC7 as explained hereabove. This region contains three HpaII fragments, one of which carries the XmaIII site. The isolated PstI-BgIII fragment was therefore digested with HpaII and the products ligated into the PstI-BgIII cleaved pJRD133 vector, using the method of Heusterspreute and Davison 1983 (Gene, in press). The resulting plasmid pJRD136 has a deletion of all three HpaII fragments (127 bp) and contains a unique XmaIII site in the tet^R region.

The plasmid pJRD136 has unique sites for virtually all commonly used restriction enzymes recognizing a 6 bp sequence but lacks sites for XhoI. A DNA linker carrying a XhoI site was therefore introduced into the Aval site of pJRD136 (this site is not being particularly useful for cloning since Aval has four possible recognition sites). This was accomplished by repair of Aval cleaved pJRD136 using DNA polymerase Kleenow fragment followed by blunt-end ligation of the linker to give pJRD139. This procedure destroys the original Aval site (however Aval also cleaves the new XhoI site).

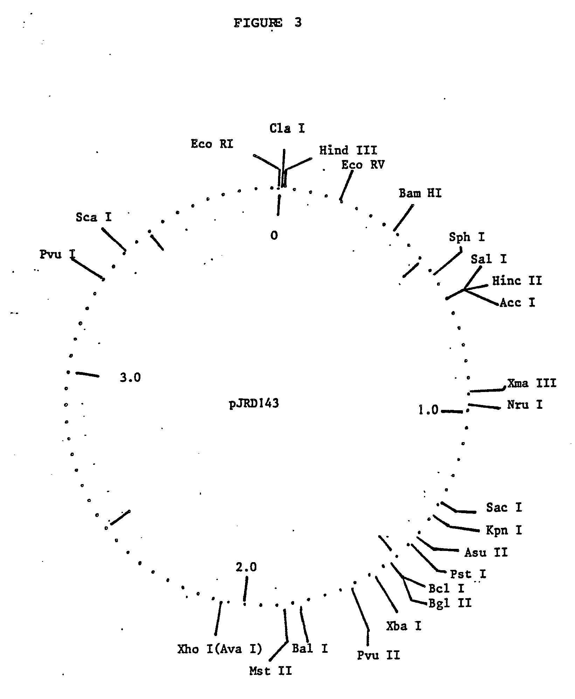

Finally an unwanted region of about 280 bp (containing an unwanted ScaI site) between the end of the tet^R gene and the SacI site was removed. The small SalI-KpnI fragment of

pJRD136 was separated from the large *Sal*I-*Kpn*I fragment and subdigested by *Taq*I and *Rsa*I followed by re-ligation to the large fragment using the techniques of Nilson and Magnusson (1983) and Heusterspreute and Davison (1983). This deletion removes the 4 terminal amino acids codons of the *tet*[R] gene and replaces them by a single amino acid codon immediately followed by a nonsense codon. The resulting plasmid (pJRD140) shows normal resistance to tetracycline. The deletion was then transferred to plasmid pJRD139 by replacing the region *Hind*III-*Pst*I resulting in plasmid pJRD143 (see Fig. 3) which contains a unique *Sca*I site.

Example 2
pJRD144—a cosmid variant of pJRD143 (see Figure 2)

The vector pJRD143 is a small multicopy plasmid very useful for the cloning of small DNA molecules (<10 kb). An intrinsic difficulty with such vectors is that they do not transform particularly well (2×10⁵ clones/µg of DNA) and that this transformation becomes increasingly inefficient when they carry large DNA inserts. Because of this large numbers of clones must be screened for a desired gene and the successful cloning of the desired gene is less likely because of the preferential cloning of small DNA fragments. A solution to these difficulties is the use of cosmids which are small plasmids containing the *cos* sites of bacteriophage λ required for packaging of DNA into λ capsids. Cosmids permit the high efficiency transfer of very large DNA molecules to *E. coli* where they are maintained as plasmids. The *cos* region of λ, contained on the 400 bp *Pst*I-*Pst*I fragment of MUA 10 (Meyerowits *et al.*, 1980) was therefore introduced into pJRD143 at the unique *Pst*I site. The resulting cosmid pJRD144 combines the restriction site bank and high copy number advantages of pJRD143 with the cosmid properties of MUA10 outlined above. It allows, all of the sites of pJRD143 to be used for cloning (except *Pst*I) and is particularly efficient for the subsequent subcloning and deletion analysis of the large cloned DNA fragment.

Example 3
Construction of an *E. coli*-yeast shuttle plasmid

*Saccharomyces cerevisiae* is an important organism for both fundamental and applied science and plasmids have been constructed that are able to replicate in both *S. cerevisiae* and in *E. coli*, using the origin of replication of the yeast 2 µ plasmid and the origin of replication of pBR322 respectively (Beggs 1981). Such yeast vectors would benefit by the introduction of more unique cloning sites such as are found in pJRD143.

Modification of the replication segment
The vector pJDB207 comprises three different DNA segments: a fragment of yeast plasmid 2 µ, responsible for DNA replication in yeast; the LEU 2 gene of yeast permitting plasmid selection and maintainance in a LEU 2⁻ yeast auxotroph; the plasmid pAT153, a deletion derivative of pBR322 (Twigg and Sherratt, 1980) resembling pBR327. The region 2 µ-LEU 2 requires little modification, since it has relatively few restriction sites and these are located in regions essential for either selection or replication.

The *Xba*I site in pJDB207 is located in the origin of replication for the 2 µ plasmid. Said *Xba*I site was removed by end-filling with DNA polymerase I followed by blunt-end ligation and transformation. The resulting plasmid (pJDB207 *Xba*I°) lacks the *Xba*I site but is unchanged in its ability to transform and replicate in yeast.

Insertion of the restriction site bank into pJDB207 *Xba*I°

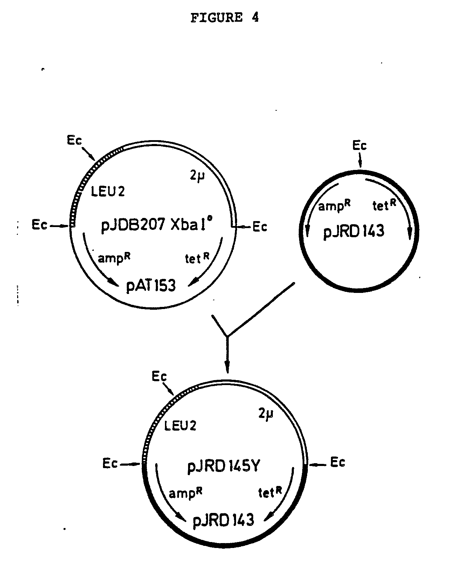

Thereafter the entire plasmid pJRD143 was inserted into pJDB207 *Xba*I° by *Eco*RI digestion and ligation, thus replacing exactly the pAT153 plasmid. The resulting plasmid pJRD145Y (Figure 4) has all of the unique restriction sites of pJRD143 except *Kpn*I which is now double and *Eco*RI which is now triple. At the same time the plasmid retains the unique *Hpa*I and *Bst*EII sites of pJDB207.

Example 4
Construction of an *E. coli*-yeast shuttle cosmid

The construction of this cosmid was achieved replacing the *Sal*I-*Bgl*II region of pJRD145Y by that of pJRD144 thereby introducing the λ *cos* site. This cosmid pJRD146Y has all of the combined advantages of pJRD144 and pJRD145Y in that it is able to grow and to be selected to both *E. coli* and yeast and it can be packaged in bacteriophage λ capsids so that the efficiency of cloning in *E. coli* is very high.

Example 5
Construction of a wide host range vector incorporating the restriction site bank

The replication of plasmids based solely upon the pMBI replicon (e.g. pMB9, pBR322 and all of their derivatives including those described above) is restricted to *E. coli* and its close relatives, so that cloning in other bacterial species is impossible. In contrast other plasmids are known that are able to replicate in a wide variety of gram negative bacteria including *Escherichia coli, Pseudomonas aeruginosa, Pseudomonas putida, Agrobacterium tumifaciens, Rhijobium meloti, Acetobacter xylinium, Azotobacter vinelandii, Alicagenes eutrophus, Rhodopseudomonas spheroides, Methylophilus methylotrophus* etc... The most promising of those plasmids as a cloning vector is RSF1010 which has a high copy number and relatively small size, but suffers the disadvantage of having very few unique restriction sites. (Nagahari and Sakaguchi, 1978). Accordingly the non-essential *Eco*RI-*Pst*I region containing the gene for resistance to sulphonamides was replaced by the *Eco*RI-*Xho*I region of pJRD143, this operation being facilitated by the addition of a *Xho*I linkers at the *Pst*I site of RSF 1010. The resulting plasmid (pJRD202 P) contains the entire restriction site bank of pJRD143 and is resistant to tetracycline and streptomycin. Most of the restriction sites are unique except *Sac*I.

**Claims**

1. A plasmid vector wherein a restriction site bank b1 derived from the *Hind*III-*Ava*I region of the bacteriophage T5 *Hind*III L fragment containing unique sites for the restriction enzymes *Hind*III, *Sac*I, *Kpn*I, *Asu*II, *Pst*I, *Bgl*II, *Xba*I, *Hinc*II, *Pvu*II, *Bal*I, *Mst*II and *Ava*I, arranged in the indicated order is attached to the *Ava*I-*Hind*III region of pBR322-related plasmids comprising a replication origin and the *amp*$^R$ gene as a selective marker.

2. A plasmid vector according to claim 1, which comprises the restriction site bank b2 resulting from the addition of two cloning sites *Xma*III and *Bcl*I to bank b1, thus containing unique sites for the restriction enzymes *Hind*III, *Sac*I, *Kpn*I, *Asu*II, *Pst*I, *Xma*III, *Bcl*I, *Bgl*II, *Xba*I, *Hinc*II, *Pvu*II, *Bal*I, *Mst*II and *Ava*I, arranged in the indicated order.

3. A plasmid vector wherein a restriction site bank b3 resulting from the addition of further sites for the restriction enzymes *Eco*RI, *Cla*I, *Hind*III, *Eco*RV, *Bam*HI, *Sph*I, *Sal*I(*Acc*I, *Hinc*II), *Xma*III and *Nru*I, arranged in the indicated order, to bank b1 is attached to the *Ava*I-*Eco*RI region of pBR322-related plasmids comprising a replication origin and the *amp*$^R$ gene as a selective marker.

4. A plasmid vector according to claim 2, which comprises the restriction site bank b4 resulting from the addition of sites for the restriction enzymes *Eco*RI, *Cla*I, *Hind*III, *Eco*RV, *Bam*HI, *Sph*I, *Sal*I(*Acc*I, *Hinc*II), *Xma*III and *Nru*I, arranged in the indicated order, to bank b2.

5. A plasmid vector according to claim 3, which comprises the restriction site bank b5 resulting from the removal of one *Hinc*II site from restriction site bank b3.

6. A plasmid vector according to claim 4, which comprises a restriction site bank b6 resulting from the removal of one *Hinc*II site from restriction site bank b4.

7. A plasmid vector according to claim 4, which comprises a restriction site bank b7 resulting from the removal of one *Xma*III site from restriction site bank b4.

8. A plasmid vector according to claim 6, which comprises a restriction site bank b8 resulting from the removal of one *Xma*III site from restriction site bank b6.

9. A plasmid vector according to claims 1 or 2 wherein the restriction site bank contains a *Xho*I site fused to the *Ava*I end thereof.

10. A plasmid vector according to any one of claims 3 to 8 wherein the restriction site bank contains a *Xho*I site fused to the *Ava*I end thereof.

11. A plasmid vector according to claims 1, 2 or 9 wherein the restriction site bank is attached to a modified version of the *Ava*I-*Hind*III region of plasmid pBR327 including mutations which increase copy number and mutations which eliminate *Pst*I and *Hinc*II sites from the *amp*$^R$ gene without destroying β-lactamase activity.

12. A plasmid vector according to claim 1 wherein the restriction site bank is attached to a modified version of the *Ava*I-*Eco*RI region of plasmid pBR327 including mutations which increase copy number and mutations which eliminate the *Pst*I and *Hinc*II sites from the *amp*$^R$ gene without destroying β-lactamase activity.

13. A plasmid vector according to claim 3 resulting from the removal of the *Sca*I site from the restriction site bank, by deletion of a 7280 bp fragment between the *tet*$^R$ gene and the *Sac*I site, leaving a unique *Sca*I site in the *amp*$^R$ gene.

14. A plasmid vector according to any one of the preceding claims which comprises a cosmid sequence in addition to a restriction site bank defined in any one of the preceding claims.

15. A yeast plasmid according to any one of the preceding claims using the origin of replication of yeast 2 μ plasmid, comprising a restriction site bank defined in any one of the preceding claims.

16. A process for the construction of a plasmid vector with the restriction site bank b3 comprising unique sites for the restriction enzymes *Hind*III, *Sac*I, *Kpn*I, *Asu*II, *Pst*I, *Bgl*II, *Xba*I, *Hinc*II, *Pvu*II, *Bal*I, *Mst*II, *Ava*I (site bank b1) and the following additional sites for restriction enzymes *Eco*RI, *Cla*I, *Hind*III, *Eco*RV, *Bam*HI, *Sph*I, *Sal*I(*Acc*I, *Hinc*II) *Xma*III and *Nru*I, which comprises the steps of:

a) deleting the segment between the two *Xba*I sites in the *Hind*III-*Ava*I region of the bacteriophage T5 *Hind*III L fragment in order to generate a single *Xba*I site; and

b) deleting the 1250 bp segment between the two *Bgl*II sites present in the same region in order to generate a single *Bgl*II site and simultaneously remove redundant *Pvu*II site;

c) fusing the *Hind*III end of restriction site bank b1 with the *Ava*I end of the *Eco*RI-*Ava*I fragment from plasmid pBR322.

17. A process for the construction of a plasmid vector with the restriction site bank b4 according to claim 16 wherein the 910 bp *Pst*I-*Bgl*II segment of bank b1 is replaced by a 210 bp *Pst*I-*Bgl*II fragment from plasmid pKC7, thus removing a part of the DNA material, present in restriction site bank b1, and resulting in a restriction site bank b2 having additional sites for restriction enzymes *Xma*III and *Bcl*I.

18. A process according to claim 16 wherein the 910 bp *Pst*I-*Bgl*II segment of bank b3 is replaced by 210 bp *Pst*I-*Bgl*II fragment from plasmid pKC7, thus removing a part of the DNA material present in restriction site bank b3, and resulting in restriction site bank b4 having additional sites for the restriction enzymes *Xma*III and *Bcl*I.

19. A process according to claim 16 which comprises the steps of:

a) deleting the *Hinc*II site in the *Bgl*II-*Ava*I

region of restriction site bank b1 while retaining adjacent *Xba*I and *Pvu*II sites, and

b) substituting the resulting deleted region for the *Bgl*II-*Ava*I region of restriction site bank b3, thus resulting in a restriction site bank b5.

20. A process according to claims 17 or 18 which comprises the steps of:

a) deleting the *Hinc*II site in the *Bgl*II-*Ava*I region of restriction site bank b1 or b2 while retaining adjacent *Xba*I and *Pvu*II sites, and

b) substituting the resulting deleted region for the *Bgl*II-*Ava*I region of the restriction site bank obtained according to claims 7 or 8, thus resulting in a restriction site bank b6.

21. A process according to claim 19, wherein the 910 bp *Pst*I-*Bgl*II segment of bank b5 is replaced by 210 bp *Pst*I-*Bgl*II fragment from plasmid pKC7, thus removing a part of the DNA material present in restriction site bank b5, and introducing *Xma*III and *Bcl*I restriction sites.

22. A process according to claims 17 or 18 which comprises deleting the *Xma*III restriction site present in the 210 bp *Pst*I-*Bgl*II fragment from plasmid pKC7, thus resulting in a restriction site bank b7 comprising a unique site for the restriction enzyme *Xma*III.

23. A process according to claims 20 or 21, which comprises deleting the *Xma*III restriction site present in the 210 bp *Pst*I-*Bgl*II fragment from plasmid pKC7, thus resulting in a restriction site bank b8 comprising unique sites for the restriction enzymes *Eco*RI, *Cla*I, *Hind*III, *Eco*RV, *Bam*HI, *Sph*I, *Sal*I(*Acc*I, *Hinc*II), *Xma*III, *Nru*I, *Sac*I, *Kpn*I, *Asu*II, *Pst*I, *Bcl*I, *Bgl*II, *Xba*I, *Pvu*II, *Bal*I, *Mst*II and *Ava*I.

24. A process according to claim 22 which comprises the steps of:

a) deleting the *Hinc*II site in the *Bgl*II-*Ava*I region of restriction site banks b1 or b2 while retaining adjacent *Xba*I and *Pvu*II sites, and

b) substituting the resulting deleted region for the *Bgl*II-*Ava*I region of restriction site bank b7, thus resulting in a restriction site bank b8.

25. A process which comprises fusing a *Xho*I site to the *Ava*I end of a restriction site bank obtained according to any one of claims 16 to 24.

26. A microorganism or cell culture transformed with a plasmid according to any one of claims 1 to 13.

27. *E. coli* MM294, transformed with plasmid pJRD143 (CBS 396-83).

28. A method for the cloning of a selected DNA material in a microorganism which comprises inserting said DNA material into one of the restriction sites present in a plasmid according to claims 1 to 15, this restriction site being selected so as to avoid cleavage of said DNA material by the restriction enzyme corresponding to the selected site and transforming said microorganism with the resulting recombinant plasmid.

29. A method for the production of a selected polypeptide which comprises inserting the DNA material coding for this polypeptide into one of the restriction sites present in a plasmid according to claims 1 to 15, this restriction site being selected so as to avoid cleavage of said coding DNA material by the restriction enzyme corresponding to the selected site, transforming a microorganism with the resulting recombinant plasmid and culturing the transformed microorganism to produce the selected polypeptide.

30. A method according to claim 29 wherein transcription of the coding DNA material is ensured by inserting an appropriate functional sequence in another selected restriction site upstream to the coding material with respect to the direction of transcription.

**Patentansprüche**

1. Plasmidvektor, worin eine Restriktionsstellenbank b1, die sich von der *Hind*III-*Ava*I-Region des Bakteriophagen T5 *Hind*III-L-Fragmentes ableitet und einmalige, in der angegebenen Reihenfolge angeordnete Stellen für die Restriktionsenzyme *Hind*III, *Sac*I, *Kpn*I, *Asu*II, *Pst*I, *Bgl*II, *Xba*I, *Hinc*II, *Pvu*II, *Bal*I, *Mst*II und *Ava*I enthält, mit der *Ava*I-*Hind*III-Region von mit PBR322 verwandten Plasmiden, die einen Replikationsursprung und das amp^R-Gen als Selektionsmarker umfassen, verbunden ist.

2. Plasmidvektor nach Anspruch 1, der die Restriktionsstellenbank b2 umfaßt, die durch Hinzufügen von zwei Klonierungsstellen *Xma*III und *Bcl*I an Bank b1 entsteht und so einmalige Stellen für die Restriktionsenzyme *Hind*III, *Sac*I, *Kpn*I, *Asu*II, *Pst*I, *Xma*III, *Bcl*I, *Bgl*II, *Xba*I, *Hinc*II, *Pvu*II, *Bal*I, *Mst*II und *Ava*I, angeordnet in der angegebenen Reihenfolge, enthält.

3. Plasmidvektor, worin die Restriktionsstellenbank b3, die durch Hinzufügen weiterer, in der angegebenen Reihenfolge angeordneter Stellen für die Restriktionsenzyme *Eco*RI, *Cla*I, *Hind*III, *Eco*RV, *Bam*HI, *Sph*I, *Sal*I(*Acc*I, *Hinc*II), *Xma*III und *Nru*I an Bank b1 entsteht, mit der *Ava*I-*Eco*RI-Region von mit pBR322 verwandten Plasmiden, die einen Replikationsursprung und das amp^R-Gen als Selektionsmarker umfassen, verbunden ist.

4. Plasmidvektor nach Anspruch 2, der die Restriktionsstellenbank b4 umfaßt, die durch Hinzufügen von Stellen für die Restriktionsenzyme *Eco*RI, *Cla*I, *Hind*III, *Eco*RV, *Bam*HI, *Sph*I, *Sal*I(*Acc*I, *Hinc*II), *Xma*III und *Nru*I, angeordnet in der angegebenen Reihenfolge, an Bank b2 entsteht.

5. Plasmidvektor nach Anspruch 3, der die Restriktionsstellenbank b5 umfaßt, die durch Entfernung einer *Hinc*II-Stelle aus der Restriktionsstellenbank b3 entsteht.

6. Plasmidvektor nach Anspruch 4, der eine Restriktionsstellenbank b6 umfaßt, die durch Entfernung einer *Hinc*II-Stelle aus der Restriktionsstellenbank b4 entsteht.

7. Plasmidvektor nach Anspruch 4, der eine Restriktionsstellenbank b7 umfaßt, die durch Entfernung einer *Xma*III-Stelle aus der Restriktionsstellenbank b4 entsteht.

8. Plasmidvektor nach Anspruch 6, der eine Restriktionsstellenbank b8 umfaßt, die durch Entfernung einer *Xma*III-Stelle aus Restriktionsstellenbank b6 entsteht.

9. Plasmidvektor nach Anspruch 1 oder 2, worin die Restriktionsstellenbank eine mit ihrem *Ava*I-Ende fusionierte *Xho*I-Stelle enthält.

10. Plasmidvektor nach irgendeinem der Ansprüche 3 bis 8, worin die Restriktionsstellenbank eine mit ihrem *Ava*I-Ende fusionierte *Xho*I-Stelle enthält.

11. Plasmidvektor nach Anspruch 1, 2 oder 9, worin die Restriktionsstellenbank mit einer modifizierten Version der *Ava*I-*Hind*III-Region von Plasmid pBR327 verbunden ist, das außerdem Mutationen, die die Kopienzahl erhöhen, und Mutationen, die die *Pst*I- und *Hinc*II-Stellen aus dem amp$^R$-Gen ohne Zerstörung und β-Lactamase-Aktivität eliminieren, besitzt.

12. Plasmidvektor nach Anspruch 1, worin die Restriktionsstellenbank mit einer modifizierten Version der *Ava*I-*Eco*RI-Region von Plasmid pBR327 verbunden ist, das außerdem Mutationen, die die Kopienzahl erhöhen, und Mutationen, die die *Pst*I- und *Hinc*II-Stellen aus dem amp$^R$-Gen ohne Zerstörung der β-Lactamase-Aktivität eliminieren, besitzt.

13. Plasmidvektor nach Anspruch 3, der durch Entfernung der *Sca*I-Stelle aus der Restriktionsstellenbank durch Deletion eines 280 bp Fragmentes zwischen dem tet$^R$-Gen und der *Sac*I-Stelle entsteht, wobei eine einzige *Sca*I-Stelle im amp$^R$-Gen verbleibt.

14. Plasmidvektor nach irgendeinem der vorhergehenden Ansprüche, der zusätzlich zu einer in irgendeinem der vorhergehenden Ansprüche definierten Restriktionsstellenbank eine Cosmid-Sequenz umfaßt.

15. Hefeplasmid nach irgendeinem der vorhergehenden Ansprüche unter Verwendung des Replikationsursprungs des 2 µ-Plasmids von Hefe, das eine in irgendeinem der vorhergehenden Ansprüche definierte Restriktionsstellenbank umfaßt.

16. Verfahren zur Konstruktion eines Plasmidvektors mit der Restriktionsstellenbank b3 mit einmaligen Stellen für die Restriktionsenzyme *Hind*III, *Sac*I, *Kpn*I, *Asu*II, *Pst*I, *Bgl*II, *Xba*I, *Hinc*II, *Pvu*II, *Bal*I, *Mst*II, *Ava*I (Bank b1) und den folgenden zusätzlichen Stellen für die Restriktionsenzyme *Eco*RI, *Cla*I, *Hind*III, *Eco*RV, *Bam*HI, *Sph*I, *Sal*I(*Acc*I, *Hinc*II), *Xma*III und *Nru*I, das die Stufen umfaßt:

a) Deletion des Segmentes zwischen den beiden *Xba*I-Stellen in der *Hind*III-*Ava*I-Region des Bakteriophagen T5 *Hind*III-L-Fragmentes zur Bildung einer einzigen *Xba*I-Stelle und

b) Deletion des 1250 bp Segmentes zwischen den beiden *Bgl*II-Stellen, die sich in der gleichen Region befinden, zur Bildung einer einzigen *Bgl*II-Stelle und gleichzeitiger Entfernung überzähliger *Pvu*II-Stelle,

c) Fusion des *Hind*III-Endes der Restriktionsstellenbank b1 mit dem *Ava*I-Ende des *Eco*RI-*Ava*I-Fragmentes aus dem Plasmid pBR322.

17. Verfahren zur Konstruktion eines Plasmidvektors mit der Restriktionsstellenbank b4 nach Anspruch 16, worin das 910 bp *Pst*I-*Bgl*II-Segment von Bank b1 durch ein 210 bp *Pst*I-*Bgl*II-Fragment aus Plasmid pKC7 ersetzt wird, wodurch ein Teil des in Restriktionsstellenbank b1 befindlichen DNA-Materials entfernt wird und eine Restriktionsstellenbank b2 mit zusätzlichen Stellen für die Restriktionsenzyme *Xma*III und *Bcl*I entsteht.

18. Verfahren nach Anspruch 16, worin das 910 bp *Pst*I-*Bgl*II-Segment von Bank b3 durch das 210 bp *Pst*I-*Bgl*II-Fragment aus Plasmid pKC7 ersetzt wird, wodurch ein Teil des in Restriktionsstellenbank b3 befindlichen DNA-Materials entfernt wird, wobei Restriktionsstellenbank b4 mit zusätzlichen Stellen für die Restriktionsenzyme *Xma*III und *Bcl*I entsteht.

19. Verfahren nach Anspruch 16, das die Stufen umfaßt:

a) Deletion der *Hinc*II-Stelle in der *Bgl*II-*Ava*I-Region von Restriktionsstellenbank b1, wobei die benachbarten *Xba*I- und *Pvu*II-Stellen erhalten bleiben, und

b) Ersetzen der *Bgl*II-*Ava*I-Region der Restriktionsstellenbank b3 durch die resultierende deletierte Region, wobei eine Restriktionsstellenbank b5 entsteht.

20. Verfahren nach Anspruch 17 oder 18, das die Stufen umfaßt:

a) Deletion der *Hinc*II-Stelle in der *Bgl*II-*Ava*I-Region von Restriktionsstellenbank b1 oder b2, wobei die benachbarten *Xba*I- und *Pvu*II-Stellen erhalten bleiben, und

b) Ersetzen der *Bgl*II-*Ava*I-Region der gemäß Anspruch 7 oder 8 erhaltenen Restriktionsstellenbank durch die resultierende deletierte Region, wobei eine Restriktionsstellenbank b6 entsteht.

21. Verfahren nach Anspruch 19, worin das 910 bp *Pst*I-*Bgl*II-Segment von Bank b5 durch das 210 bp *Pst*I-*Bgl*II-Fragment aus Plasmid pKC7 ersetzt wird, wodurch ein Teil des in Restriktionsstellenbank b5 befindlichen DNA-Materials entfernt wird und *Xma*III- und *Bcl*I-Restriktionsstellen eingeführt werden.

22. Verfahren nach Anspruch 17 oder 18, das die Deletion der *Xma*III-Restriktionsstelle umfaßt, die sich in 210 bp *Pst*I-*Bgl*II-Fragment aus Plasmid pKC7 befindet, woraus eine Restriktionsstellenbank b7 resultiert, die eine einmalige Stelle für das Restriktionsenzym *Xma*III umfaßt.

23. Verfahren nach Anspruch 20 oder 21, das die Deletion der *Xma*III-Restriktionsstelle umfaßt, die sich im 210 bp *Pst*I-*Bgl*II-Fragment aus Plasmid pKC7 befindet, wodurch eine Restriktionsstellenbank b8 entsteht, die einmalige Stellen für die Restriktionsenzyme *Eco*RI, *Cla*I, *Hind*III, *Eco*RV, *Bam*HI, *Sph*I, *Sal*I(*Acc*I, *Hinc*II), *Xma*III, *Nru*I, *Sac*I, *Kpn*I, *Asu*II, *Pst*I, *Bcl*I, *Bgl*II, *Xba*I, *Pvu*II, *Bal*I, *Mst*II und *Ava*I umfaßt.

24. Verfahren nach Anspruch 22, das die Stufen umfaßt:

a) Deletion der *Hinc*II-Stelle in der *Bgl*II-*Ava*I-Region der Restriktionsstellenbanken b1 oder b2, wobei die benachbarten *Xba*I- und *Pvu*II-Stellen erhalten bleiben, und

b) Ersetzen der *Bgl*II-*Ava*I-Region von Restriktionsstellenbank b7 durch die resultierende, deletierte Region, wodurch eine Restriktionsstellenbank b8 entsteht.

25. Verfahren, das die Fusion einer *Xho*I-Stelle mit dem *Ava*I-ende einer Restriktionsstellenbank, die nach irgendeinem der Ansprüche 16 bis 24 erhalten worden ist, umfaßt.

26. Mikroorganismus oder Zellkultur, der bzw. die mit einem Plasmid nach irgendeinem der Ansprüche 1 bis 13 transformiert ist.

27. *E. coli* MM294, transformiert mit Plasmid pJRD143 (CBS 396-83).

28. Verfahren zum Klonen eines ausgewählten DNA-Materials in einem Mikroorganismus, das die Insertion der DNA-Materials in eine der in einem Plasmid nach den Ansprüchen 1 bis 15 vorhandenen Restriktionsstellen und das Transformieren des Mikroorganismus mit dem resultierenden, rekombinanten Plasmid umfaßt, wobei die Restriktionsstelle so gewählt wurde, daß eine Spaltung des DNA-Materials durch das Restriktionsenzym entsprechend der gewählten Stelle vermieden wird.

29. Verfahren zur Produktion eines ausgewählten Polypeptids, das die Insertion des das Polypeptid kodierenden DNA-Materials in eine der in einem Plasmid nach den Ansprüchen 1 bis 15 vorhandenen Restriktionsstellen, das Transformieren eines Mikroorganismus mit dem resultierenden, rekombinanten Plasmid und das Züchten des transformierten Mikroorganismus zur Produktion des ausgewählten Polypeptids umfaßt, wobei die Restriktionsstelle so gewählt wurde, daß eine Spaltung des kodierenden DNA-Materials durch das Restriktionsenzym entsprechend der gewählten Stelle vermieden wird.

30. Verfahren nach Anspruch 29, worin die Transkription des kodierenden DNA-Materials gesichert wird, indem man eine geeignete funktionelle Sequenz in einer anderen ausgewählten Restriktionsstelle stromaufwärts des kodierenden Materials, bezogen auf die Richtung der Transkription, inseriert.

**Revendications**

1. Vecteur plasmidique dans lequel une banque b1 de sites de restriction, dérivant de la région *Hind*III-*Ava*I du fragment *Hind*III-L du bactériophage T5, cette région contenant des sites uniques pour les enzymes de restriction *Hind*III, *Sac*I, *Kpn*I, *Asu*II, *Pst*I, *Bgl*II, *Xba*I, *Hinc*II, *Pvu*II, *Bal*I, *Mst*II et *Ava*I, ces sites étant disposés dans l'ordre indiqué, est fixée à la région *Ava*I-*Hind*III des plasmides apparentés à pBR322, cette région comprenant une origine de réplication ainsi que le gène *amp*R comme marqueur sélectif.

2. Vecteur plasmidique selon la revendication 1, ce vecteur contenant la banque b2 de sites de restriction résultant de l'addition de deux sites de clonage *Xma*III et *Bcl*I à la banque b1, ce vecteur contenant ainsi des sites uniques pour les enzymes de restriction *Hind*III, *Sac*I, *Kpn*I, *Asu*II, *Pst*I, *Xma*III, *Bcl*I, *Bgl*II, *Xba*I, *Hinc*II, *Pvu*II, *Bal*I, *Mst*II et *Ava*I, ces sites étant disposés dans l'ordre indiqué.

3. Vecteur plasmidique dans lequel une banque b3 de sites de restriction résultant de l'addition à la banque b1 d'autres sites pour les enzymes de restriction *Eco*RI, *Cla*I, *Hind*III, *Eco*RV, *Bam*HI, *Sph*I, *Sal*I, (*Acc*I, *Hinc*II), *Xma*III et *Nru*I, ces sites étant disposés dans l'ordre indiqué, est fixée à la région *Ava*I-*Eco*RI des plasmides apparentés à pBR322, cette région contenant une origine de réplication ainsi que le gène *amp*R comme marqueur sélectif.

4. Vecteur plasmidique selon la revendication 2, ce vecteur contenant la banque b4 de sites de restriction résultant de l'addition des sites pour les enzymes de restriction *Eco*RI, *Cla*I, *Hind*III, *Eco*RV, *Bam*HI, *Sph*I, *Sal*I (*Acc*I, *Hinc*II), *Xma*III et *Nru*I, ces sites étant disposés dans l'ordre indiqué, à la banque b2.

5. Vecteur plasmidique selon la revendication 3, ce vecteur contenant la banque b5 de sites de restriction résultant de l'élimination d'un site *Hinc*II de la banque b3 de sites de restriction.

6. Vecteur plasmidique selon la revendication 4, ce vecteur contenant une banque b6 de sites de restriction résultant de l'élimination d'un site *Hinc*II de la banque b4 de sites de restriction.

7. Vecteur plasmidique selon la revendication 4, ce vecteur contenant une banque b7 de sites de restriction résultant de l'elimination d'un site *Xma*III de la banque b4 de sites de restriction.

8. Vecteur plasmidique selon la revendication 6, ce vecteur contenant une banque b8 de sites de restriction résultant de l'élimination d'un site *Xma*III de la banque b6 de sites de restriction.

9. Vecteur plasmidique selon la revendication 1 ou 2, dans lequel la banque de sites de restriction contient un site *Xho*I fusionné à l'extrémité *Ava*I de celle-ci.

10. Vecteur plasmidique selon l'une quelconque des revendications 3 à 8, dans lequel la banque de sites de restriction contient un site *Xho*I fusionné à l'extrémité *Ava*I de celle-ci.

11. Vecteur plasmidique selon les revendications 1, 2 ou 9, dans lequel la banque de sites de restriction est fixée à une version modifiée de la région *Ava*I-*Hind*III du plasmide pBR327, cette version comprenant des mutations qui ont pour effet d'augmenter le nombre de copies, ainsi que des mutations qui ont pour effet d'éliminer les sites *Pst*I et *Hinc*II du gène *amp*R, sans pour autant détruire l'activité β-lactamasique.

12. Vecteur plasmidique selon la revendication 1, dans lequel la banque de sites de restriction est fixée à une version modifiée de la région *Ava*I-*Eco*RI du plasmide pBR327, cette version comprenant des mutations qui ont pour effet d'augmenter le nombre de copies, ainsi que des mutations qui ont pour effet d'éliminer les sites *Pst*I et *Hinc*II du gène *amp*R sans pour autant détruire l'activité β-lactamasique.

13. Vecteur plasmidique selon la revendication 3, ce vecteur résultant de l'élimination du site *Sca*I de la banque de sites de restriction par suppression d'un fragment de 280 pb compris entre le gène *tet*R et le site *Sac*I, ce qui laisse un site *Sac*I unique dans le gène *amp*R.

14. Vecteur plasmidique selon l'une quelconque des revendications précédentes, ce vecteur contenant une séquence cosmidique en plus d'une banque de sites de restriction telle que définie dans l'une quelconque des revendications précédentes.

15. Plasmids de levure, selon l'une quelconque des revendications précédentes, ce plasmide comprenant l'origine de réplication de plasmide 2 μ de levure ainsi qu'une banque de sites de restriction telle que définie dans l'une quelconque des revendications précédentes.

16. Procédé de construction d'un vecteur plasmidique contenant la banque b3 de sites de restriction, celle-ci comprenant des sites uniques pour les enzymes de restriction *Hind*III, *Sac*I, *Kpn*I, *Asu*II, *Pst*I, *Bg*III, *Xba*I, *Hinc*II, *Pvu*II, *Bal*I, *Mst*II, *Ava*I (banque b1 de sites) et des sites supplémentaires pour les enzymes de restriction *Eco*RI, *Cla*I, *Hind*III, *Eco*RV, *Bam*HI, *Sph*I, *Sal*I (*Acc*I, *Hinc*II), *Xma*III et *Nru*I, ce procédé comprenant les étapes consistant à:

a) supprimer le segment compris entre les deux sites *Xba*I situés dans la région *Hind*III-*Ava*I du fragment *Hind*III L du bactériophage T5, afin de produire un site *Xba*I unique; et

b) supprimer le segment de 1250 pb compris entre les deux sites *Bg*III présents dans la même région, afin de produire un site *Bg*III unique et, en même temps, d'éliminer le site *Pvu*II redondant;

c) fusionner l'extrémité *Hind*III de la banque b1 de sites de restriction à l'extrémité *Ava*I du fragment *Eco*RI-*Ava*I du plasmide pBR322.

17. Procédé de construction d'un vecteur plasmidique contenant la banque b4 de sites de restriction, selon la revendication 16, procédé selon lequel on remplace le segment *Pst*I-*Bg*III de 910 pb de la banque b1 par un fragment *Pst*I-*Bg*III de 210 pb du plasmide pKC7, éliminant ainsi une partie de l'ADN présent dans la banque b1 de sites de restriction, ce qui donne lieu à une banque b2 de sites de restriction contenant des sites supplémentaires pour les enzymes de restriction *Xma*III et *Bcl*I.

18. Procédé selon la revendication 16 selon lequel on remplace le segment *Pst*I-*Bg*III de 910 pb de la banque b3 par le fragment *Pst*I-*Bg*III de 210 pb du plasmide pKC7, éliminant ainsi une partie de l'ADN présent dans la banque b3 de sites de restriction, ce qui donne lieu à la banque b4 de sites de restriction contenant des sites supplémentaires pour les enzymes de restriction *Xma*III et *Bcl*I.

19. Procédé selon la revendication 16, ce procédé comprenant les étapes consistant à:

a) supprimer le site *Hinc*II se trouvant dans la région *Bg*III-*Ava*I de la banque b1 de sites de restriction, tout en maintenant les sites adjacents *Xba*I et *Pvu*II, et

b) substituer la région supprimée résultante à la région *Bg*III-*Ava*I de la banque b3 de sites de restriction, donnant ainsi lieu à une banque b5 de sites de restriction.

20. Procédé selon la revendication 17 ou 18, ce procédé comprenant les étapes consistant à:

a) supprimer le site *Hinc*II se trouvant dans la région *Bg*III-*Ava*I de la banque b1 ou b2 de sites de restriction, tout en conservant les sites adjacents *Xba*I et *Pvu*II, et

b) substituer la région supprimée résultante à la région *Bg*III-*Ava*I de la banque de sites de restriction telle qu'obtenue selon la revendication 7 ou 8, donnant lieu à une banque b6 de sites de restriction.

21. Procédé selon la revendication 19, dans lequel on remplace le segment *Pst*I-*Bal*II de 910 pb de la banque b5 par le fragment *Pst*I-*Bg*III de 210 pb du plasmide pKC7, éliminant ainsi une partie de la matière d'ADN présente dans la banque b5 de sites de restriction, tout en y insérant les sites de restriction *Xma*III et *Bcl*I.

22. Procédé selon la revendication 17 ou 18, dans lequel on supprime le site de restriction *Xma*III présent dans le fragment *Pst*I-*Bg*III de 210 pb du plasmide pKC7, ceci donnant lieu à une banque b7 de sites de restriction contenant un site unique pour l'enzyme de restriction *Xma*III.

23. Procédé selon la revendication 20 ou 21, dans lequel on supprime le site de restriction *Xma*III présent dans le fragment *Pst*I-*Bg*III de 210 pb du plasmide pKC7, ceci donnant lieu à une banque b8 de sites de restriction contenant des sites uniques pour les enzymes de restriction *Eco*RI, *Cla*I, *Hind*III, *Eco*RV, *Bam*HI, *Sph*I, *Sal*I (*Acc*I, *Hinc*II), *Xma*III, *Nru*I, *Sac*I, *Kpn*I, *Asu*II, *Pst*I, *Bal*I, *Bg*III, *Xba*I, *Pvu*II, *Bal*I, *Mst*II et *Ava*I.

24. Procédé selon la revendication 22 comprenant les étapes consistant à:

a) supprimer le site *Hinc*II se trouvant dans la région *Bg*III-*Ava*I des banques b1 ou b2 de sites de restriction, tout en maintenant les sites adjacents *Xba*I et *Pvu*II, et

b) substituer la région supprimée résultante par la région *Bg*III-*Ava*I de la banque b7 de sites de restriction, donnant ainsi lieu à une banque b8 de sites de restriction.

25. Procédé selon lequel on fusionne un site *Xho*I à l'extrémité *Ava*I d'une banque de sites de restriction obtenue selon l'une quelconque des revendications 16 à 24.

26. Micro-organisme ou culture cellulaire transformé par un plasmide selon l'une quelconque des revendications 1 à 13.

27. *E. coli* MM294 transformé par le plasmide pJRD143 (CBS 396-83).

28. Procédé de clonage d'ADN dans un micro-organisme, ce procédé comprenant les étapes consistant à insérer cet ADN dans l'un des sités de restriction présents dans un plasmide selon l'une des revendications 1 à 15, ce site de restriction étant choisi de telle sorte que l'on évite le clivage de cet ADN par l'enzyme de restriction correspondant au site choisi, et transformer ce micro-organisme via le plasmide recombinant résultant.

29. Procédé de préparation d'un polypeptide, ce procédé comprenant les étapes consistant à insérer l'ADN codant pour ce polypeptide dans l'un des sites de restriction présents dans un plasmide selon les revendications 1 à 15, ce site de restriction étant choisi afin d'éviter le clivage de cet ADN par l'enzyme de restriction corresapondant au site choisi, transformer un micro-organisme par le plasmide recombinant résultant et cultiver le micro-organisme transformé pour obtenir le polypeptide.

30. Procédé selon la revendication 29 par lequel on assure la transcription de l'ADN codant pour un polypeptide en insérant une séquence fonctionnelle appropriée dans un autre site de restriction situé en amont de la partie codante par rapport à la direction de transcription.

FIGURE 1

1

FIGURE 2A

2

FIGURE2B

FIGURE 3

FIGURE 4